# EUROPEAN PATENT APPLICATION

(11) **EP 2 180 339 A1**
(43) Date of publication of application: **28.04.2010**
(21) Application number: 09172531.7
(22) Date of filing: 08.10.2009
(51) Int. Cl.: G01S 15/00, A61B 8/08, G01P 5/24, G01S 7/52, G01S 15/58

(54) **Doppler signal processing for an enhanced spectral doppler image**

(30) Priority: 21.10.2008 KR 20080102818
(71) Applicant: MEDISON CO., LTD., Kangwon-do 250-870 (KR)
(72) Inventor: Lee, Jae Keun, 1003 Daechi-dong Gangnam-gu 135-851, Seoul (KR)
(74) Representative: Schmid, Wolfgang

(57) **Abstract**

Embodiment for processing Doppler signals for an enhanced spectral Doppler image in an ultrasound system is disclosed. The Doppler signals, which are obtained by transmitting/receiving an ultrasound signal to/from a target object, are window-processed with a plurality of windows, each of the windows having different sizes. The window-processed Doppler signals are transformed into a plurality of frequency-domain Doppler signals. The transformed Doppler signals are compounded and then a spectral Doppler image is formed based on the compound Doppler signals.

## Description

The present application claims priority from Korean Patent Application No. 10-2008-0102818 filed on October 21, 2008, the entire subject matter of which is incorporated herein by reference.

### TECHNICAL FIELD

The present disclosure generally relates to ultrasound imaging, and more particularly to Doppler signal processing to provide an enhanced spectral Doppler image.

### BACKGROUND

An ultrasound system has become an important and popular diagnostic tool since it has a wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., human organs).

Generally, the ultrasound system provides a B-mode image, which 2-dimensionally visualizes the reflectivity of ultrasound signals reflected from a target object, in a B-mode. Also, the ultrasound diagnostic system provides a spectral Doppler image, which visualizes the velocities and direction of moving objects, e.g., blood flow based on Doppler signals obtained by using the Doppler Effect, in a Doppler mode. In the Doppler mode, the ultrasound system transmits ultrasound signals into a target object at a pulse repetition frequency and receives echo signals reflected from the target object through an ultrasound probe. The ultrasound system obtains Doppler signals by computing a frequency shift between frequencies of the transmitted ultrasound signals and received ultrasound signals. The frequency of the Doppler signals, which are obtained from the target object approaching toward an ultrasound probe, may be higher than the frequency of the transmitted ultrasound signals. On the other hand, the frequency of the Doppler signals, which are obtained moving away from the ultrasound probe, may be less than the frequency of the transmitted ultrasound signals.

To form the spectral Doppler image, the ultrasound system performs window processing upon the Doppler signals by using a predetermined window, e.g., Hanning window, etc., to make discrete Doppler signals continuous. In such a case, a window size may significantly affect the temporal resolution of the Spectral Doppler image. Conventionally, the window size is set according to a Doppler scale in the spectral Doppler image, thus resulting in the lowered temporal resolution of the Spectral Doppler image.

### SUMMARY

Embodiments for Doppler signal processing are disclosed herein. In one embodiment, by way of non-limiting example, an ultrasound system comprises: a Doppler signal acquisition unit operable to transmit an ultrasound transmit beam into a target object and receive echo signals reflected from the target object, thereby forming Doppler signals; a signal processing unit operable to perform window-processing upon the Doppler signals with a plurality of windows, each of the windows having different sizes, to output a plurality of window-processed Doppler signals and transform the window-processed Doppler signals into a plurality of frequency-domain Doppler signals, the signal processing unit further being operable to compound the transformed Doppler signals to output compound Doppler signals; and an image processing unit operable to form a Spectral Doppler image based on the compound Doppler signals.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIG. 2 is a block diagram showing an illustrative embodiment of the Doppler signal acquisition unit.
FIG. 3 is a block diagram showing an illustrative embodiment of a signal processing unit.

### DETAILED DESCRIPTION OF THE PRESENT INVENTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

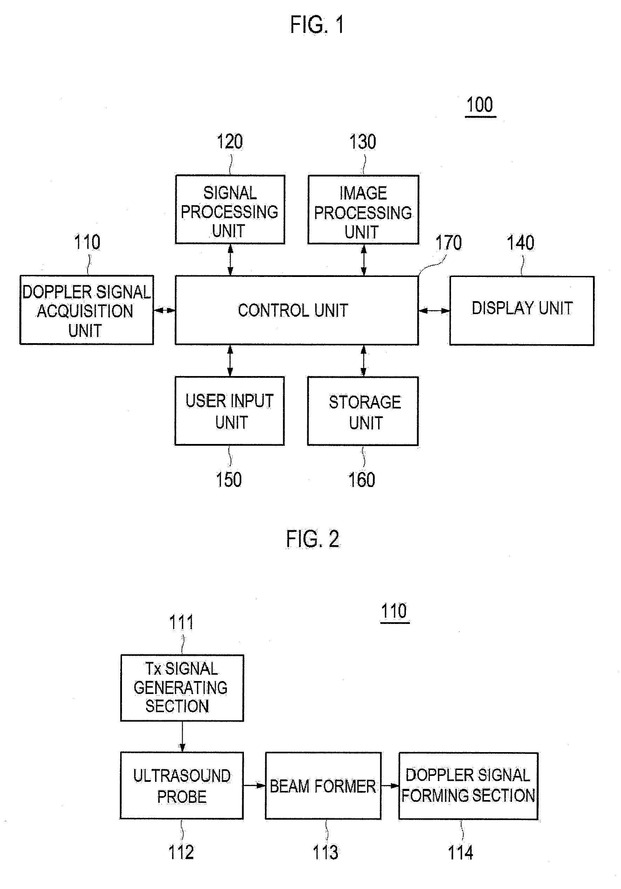

FIG. 1 is a block diagram showing an illustrative embodiment of an ultrasound system 100. As shown in FIG. 1, the ultrasound system 100 may include a Doppler signal acquisition unit 110. The Doppler signal acquisition unit 100 may be operable to transmit an ultrasound transmission (Tx) beam into a target object and receive echo signals reflected from the target object. The transmission of the ultrasound Tx beam may be performed at a preset pulse repetition frequency (PRF). The Doppler signal acquisition unit 110 may be further operable to form Doppler signals based on a frequency shift between frequencies of the ultrasound Tx beam and the echo signals by using the Doppler Effect.

FIG. 2 is a block diagram showing an illustrative embodiment of the Doppler signal acquisition unit 110. Referring to FIG. 2, the Doppler signal acquisition unit 110 may include a transmission (Tx) signal generating section 111, an ultrasound probe 112, a beam former 113 and a Doppler signal forming section 114. The ultrasound probe 112 may include an array transducer containing a plurality of elements.

The Tx signal generating section 111 may be operable to generate a plurality of Tx signals. The tx signals may be applied to the elements in the ultrasound probe 112. In one embodiment, the Tx signals may be repeatedly generated at a predetermined time intervals, i.e., at the preset PRF, in the Doppler mode of the ultrasound system 100.

The ultrasound probe 112 is coupled to the Tx signal generating section 111. The ultrasound probe 112 may be operable to convert the Tx signals into ultrasound signals to thereby transmit an ultrasound Tx beam to the target object. The transmission of the ultrasound Tx beam may be repeated according to the generation of the Tx signals. The ultrasound probe 112 may be further operable to receive echo signals reflected from the target object and convert the receive echo signals into electrical receive signals.

The beam former 113 is coupled to the ultrasound probe 112 to receive the receive signals. The beam former 113 may be operable to perform analog-to-digital conversion upon the electrical receive signals to thereby output digital receive signals. The beam former 113 may be further operable to apply delays to the digital receive signals in consideration of positions of the transducer elements and focal points, and then sum the delayed digital receive signals to thereby output a plurality of receive-focused beams.

The Doppler signal forming section 114 may be operable to form time-domain Doppler signals based on the receive-focused beams. The Doppler signals may include high-frequency Doppler signals caused by a blood flow and low-frequency Doppler signals caused by motions of a cardiac wall, a cardiac valve and the like. The low-frequency Doppler signals are generally referred to as "clutter signals." The clutter signals may have amplitudes of over 100 times than the high-frequency Doppler signals.

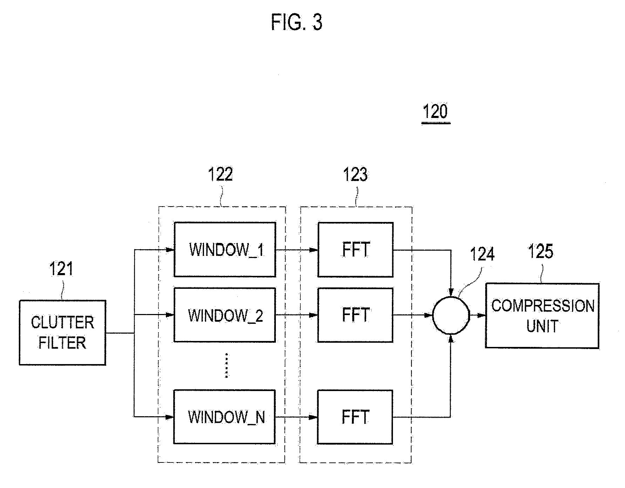

Referring back to FIG. 1, the ultrasound system 100 may further include the signal processing unit 120 coupled to the Doppler signal acquisition unit 110. As shown in FIG. 3, the signal processing unit 120 may include a clutter filter 121. The clutter filter 121 may be operable to filter the Doppler signals acquired by the Doppler signal acquisition unit 110 to remove the clutter signals therefrom. The clutter filter 121 may output clutter-removed Doppler signals.

The signal processing unit 120 may further include a window-processing section 122. The window-processing section 122 may include a plurality of window-processors WINDOW_1-WINDOW_N, which may be operable to perform window-processing upon the clutter-removed Doppler signals by using a plurality of windows such as a Hanning window, etc. In one embodiment, the windows may be set to have different window sizes. The setting of the windows will be described later. The window-processing section 122 may be operable to output a plurality of window-processed Doppler signals.

The signal processing unit 120 may further include a fast Fourier transformation (FFT) section 123. The FFT section 123 may include a plurality of transformers corresponding to the window-processors. The transformers may be operable to fast Fourier transform the plurality of window-processed Doppler signals from the time domain to the frequency domain, thereby outputting transformed Doppler signals.

The signal processing unit 120 may further include a compound section 124. The compound section 124 may be operable to compound the transformed Doppler signals, which are outputted from the transformers. In one embodiment, the signal processing unit 120 may be operable to apply a different weight to each of the transformed Doppler signals, and then compound the weighted Doppler signals to thereby output compound Doppler signals.

The signal processing unit 120 may further include a compression section 125, which may be operable to compress the compound Doppler signals. While in the illustrated embodiment the compression section 125 is configured to be placed next to the compound section 124, the configuration of the compression section 125 may not be limited thereto. The compression section 125 may be positioned between the FFT section 123 and the compound section 124.

The ultrasound system 100 may further include an image processing unit 130, which may be operable to form a spectral Doppler image based on the compressed Doppler signals by the signal processing unit 120. The ultrasound system 100 may further include a display unit 140. The display unit 140 may display the spectral Doppler image. As mentioned above, since the spectral Doppler image is formed by compounding the plurality of Doppler signals, which are window-processed with different window sizes, the enhanced temporal resolution of the spectral Doppler image may be obtained.

The ultrasound system 100 may further include a user input unit 150. The user input unit 150 may be operable to allow a user to input window setting information and compound setting information. In one embodiment, the window setting information may include selection information to select at least two windows for window processing. That is, the window-processors in the window-processing section 122 may be determined according to the window setting information. Also, the compound setting information may include information on at least two weights to be applied to the transformed Doppler signals.

The ultrasound system 100 may further include a storage unit 160. The storage unit 160 may store a mapping table in which reference window sizes are mapped to pulse repetition frequencies, as shown in the following table.

**[Table]**

| Pulse Repetition Frequency (Hz) | Reference Window Size |
|---|---|
| 1000 | 30 |
| 2000 | 32 |
| 6000 | 38 |
| 8000 | 44 |
| 23000 | 128 |

The ultrasound system 100 may further include a control unit 170. If the window setting information is inputted through the input unit 150, then the control unit 170 may be operable to control the setting of windows in the window processing section 122 based on the window setting information. The control unit 170 may be operable to extract information on a reference window size corresponding to the preset pulse repetition frequency, which are used to transmit the ultrasound Tx beam, from the storage unit 160. The control unit 170 may be further operable to control the window-processing section 122 such that a plurality of windows is set based on the extracted reference window size information. In one embodiment, the windows may be set to have different window sizes.

In one embodiment, the window setting information may further include information on the sizes of the selected windows. In such a case, the window sizes may be determined based on the size information of the windows without extracting the reference window size information from the storage unit 160.

Also, the control unit 173 may be further operable to control the setting of the transformers at the FFT section 123. That is, the control unit 173 may be operable to control the FFT section 123 such that a plurality of transformers corresponding to the set windows is set. Further, if the compound setting information is inputted through the user input unit 150, then the control unit 170 may be operable to control the FFT section 123 such that a plurality of weights to be applied to the window-processed Doppler signals are set based on the inputted compound setting information. The control 170 may be further operable to control the transmission and reception of the ultrasound signals, the signal processing of the Doppler signals, the formation of the Spectral Doppler image and the display of the Spectral Doppler image.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a Doppler signal acquisition unit for transmitting an ultrasound transmit beam into a target object and receiving echo signals reflected from the target object to form Doppler signals;
a signal processing unit for performing window-processing upon the Doppler signals with a plurality of windows, each of the windows having different sizes, to output a plurality of window-processed Doppler signals and transform the plurality of window-processed Doppler signals into a plurality of frequency-domain Doppler signals, the signal processing unit being further configured to compound the transformed Doppler signals to output compound Doppler signals; and
an image processing unit for forming a Spectral Doppler image based on the compound Doppler signals.

2. The ultrasound system of Claim 1, further comprising:
a storage unit for storing a mapping table mapping a plurality of pulse repetition frequencies to a plurality of reference window sizes;
a user input unit for allowing a user to input window setting information for setting the plurality of windows; and
a control unit for controlling the transmission of the ultrasound transmit beam at one of the pulse repetition frequencies, the control unit being further configured to extract a reference window size corresponding to the pulse repetition frequency used to transmit the ultrasound transmit beam from the mapping table stored in the storage unit and set the plurality of windows based on the window setting information and the extracted reference window size.

3. The ultrasound system of Claim 2, wherein the signal processing unit is further configured to compress the compound Doppler signals.

4. The ultrasound system of Claim 2, wherein the user input is further configured to allow the user input compound setting information for setting different weights to be applied to the transformed Doppler signals.

5. The ultrasound system of Claim 4, wherein the signal processing section is further configured to apply the weights to the transformed Doppler signals to output weighted Doppler signals and compound the weighted Doppler signals.
